# EUROPÄISCHE PATENTSCHRIFT

(11) **EP 0 598 354 B1**
(45) Veröffentlichungstag und Bekanntmachung des Hinweises auf die Patenterteilung: **09.04.1997**
(21) Anmeldenummer: 93118336.2
(22) Anmeldetag: 12.11.1993
(51) Int. Cl.: C07C 25/18, C09K 19/18, C09K 19/20, C09K 19/30, C07C 43/225, C07C 43/192, C07C 255/46, G02F 1/13

(54) **Fluorsubstituierte Tolan-Derivate und sie enthaltende flüssigkristalline Gemische**
Fluor substituted tolan derivatives and liquid crystals mixtures containing them
Dérivés du tolane substitués par du fluor et mélanges de cristaux liquides les contenant

(30) Priorität: 16.11.1992 CH 3520/92
(43) Veröffentlichungstag der Anmeldung: 25.05.1994
(73) Patentinhaber: MERCK PATENT GmbH, 64293 Darmstadt (DE)
(72) Erfinder: Buchecker, Richard, CH-8008 Zürich (CH); Schadt, Martin, CH-4411 Seltisberg (CH)

(56) Entgegenhaltungen:
- WO-A-88/07514
- DE-A- 4 105 742

## Beschreibung

Die vorliegende Erfindung betrifft neue, flüssigkristalline, mehrfach fluorierte Tolan-Derivate, deren Herstellung, flüssigkristalline Gemische, die solche Verbindungen enthalten, sowie die Verwendung dieser Verbindungen bzw. Gemische für elektro-optische Zwecke.

Flüssige Kristalle werden vor allem als Dielektrika in Anzeigevorrichtungen verwendet, da die optischen Eigenschaften solcher Substanzen durch eine angelegte Spannung beeinflusst werden können. Elektrooptische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super twisted nematic"), SBE-Zellen ("super birefringence effect") und OMI-Zellen ("optical mode interference"). Für Displays mit hohem Informationsgehalt sind in letzter Zeit neben den passiv angesteuerten, multiplexierten, besonders die aktiv angesteuerten, z.B. TFT-Zellen ("thin film transistor") wichtig geworden. Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische, photochemische und thermische Stabilität und eine gute Stabilität gegenüber elektrischen Feldern besitzen. Ferner sollten sie über einen möglichst breiten Bereich eine geeignete Mesophase aufweisen (beispielsweise für die oben genannten Zellen eine nematische bzw. eine cholesterische Phase), aber trotzdem ausreichend niedere Viskosität besitzen und in den Zellen kurze Ansprechzeiten, tiefe Schwellenspannungen und einen hohen Kontrast ermöglichen. Weitere Eigenschaften, wie die elektrische Leitfähigkeit, die dielektrische Anisotropie und die optische Anisotropie, müssen je nach Anwendungsgebiet und Zellentyp unterschiedlichen Anforderungen genügen. Beispielsweise sollten Materialien für die Zellen mit verdrillt nematischer Struktur eine möglichst hohe positive dielektrische Anisotropie und gleichzeitig eine möglichst geringe Leitfähigkeit aufweisen. Diese letztere Eigenschaft ist vorallem für TFT-Zellen von besonderer Wichtigkeit. Leider führen aber Komponenten mit höherer dielektrischer Anisotropie infolge ihres besseren Lösungsvermögens für ionische Verunreinigungen meist zu einer erhöhten Leitfähigkeit in Mischungen. Es werden daher Komponenten gesucht, die sich durch eine möglichst hohe dielektrische Anisotropie bei gleichzeitig möglichst geringer Leitfähigkeit auszeichnen. **In WO-A-88 07514 und in DE-A-4 105 742 werden Tolane Derivate als Flüssigkristall-Kompnenten beschrieben.**

Gegenstand der **vorliegenden** Erfindung sind Verbindungen der allgemeinen Formel 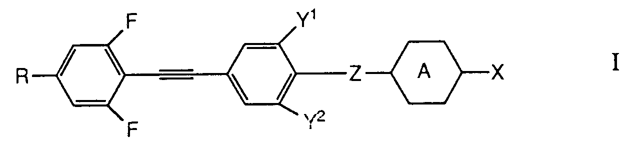 worin
- R: Alkyl, Alkoxy, Alkenyl oder Alkenyloxy mit 1 bzw. 2 bis 12 Kohlenstoffatomen bedeutet, in welchen eine CH₂-Gruppe durch Sauerstoff und/oder eines oder mehrere Wasserstoffatome durch Fluoratome ersetzt sein können, mit der Massgabe, dass zwei Sauerstoffatome nicht direkt benachbart sind;
- Y¹, Y²: unabhängig voneinander Fluor oder Wasserstoff bedeuten;
- Z: eine einfache Kovalenzbindung, -CH₂CH₂-, -OCH₂-, -CH₂O-, -(CH₂)₄-, -O(CH₂)₃-, -(CH₂)₃O-, oder, falls Ring A einen gesättigten Ring darstellt, auch die trans-Form von -(CH₂)₂CH=CH- oder -CH₂OCH=CH- bedeutet;
- A: trans-1,4-Cyclohexylen oder, gegebenenfalls mit Fluor substituiertes, 1,4-Phenylen bedeutet;
- X: Alkyl, Alkenyl, Alkoxy, Alkenyloxy oder Alkoxyalkyl mit 1 bzw. 2 bis 6 Kohlenstoffatomen oder -CH=CF₂, oder an trans-1,4-Cyclohexylen auch -CH=CHCl oder -CH=CHF, oder an 1,4-Phenylen auch Fluor, Chlor, -CF₃, -OCF₃ oder -OCHF₂ bedeutet.

Die erfindungsgemässen Verbindungen sind Flüssigkristalle mit ausgeprägter nematischen Tendenz, die in Mischungen, auch wenn sie isotrop sind, nur zu geringfügigen Klärpunktsdepressionen führen. Sie zeichnen sich durch eine hohe dielektrische Anisotropie, relativ niedrige Rotationsviskosität und daher vergleichsweise niedrige Schwellenspannungen und kurze Ansprechzeiten aus. Trotz der hohen dielektrischen Anisotropie ist die Leitfähigkeit relativ niedrig. Zudem ist der Klärpunkt der Verbindungen trotz mehrfacher lateraler Substitution überraschend hoch bei vergleichsweise niedrigem Schmelzpunkt und kleiner Schmelzenthalpie. Durch die geeignete Wahl eines gesättigten oder aromatischen Rings für A kann die relativ hohe optische Anisotropie wunschgemäss erniedrigt oder noch weiter erhöht werden.

Die erfindungsgemässen Verbindungen sind in Mischungen sehr gut und in breiten Konzentrationsbereichen löslich. Sie eignen sich besonders zur Anwendung in Mischungen, die bei tiefer Schwellenspannung eine tiefe Leitfähigkeit und gleichzeitig eine vergleichsweise hohe optische Anisotropie aufweisen sollten, wie beispielsweise für TN-, STN- oder TFT-Zellen mit geringer Schichtdicke oder mit besonders hohem Kontrast, wie sie beispielsweise für Projektionsdisplays benötigt werden.

Der Ausdruck "Alkyl, Alkoxy, Alkenyl oder Alkenyloxy mit 1 bzw. 2 bis 12 Kohlenstoffatomen, in welchen eine CH₂-Gruppe durch Sauerstoff und/oder eines oder mehrere Wasserstoffatome durch Fluoratome ersetzt sein können, mit der Massgabe, dass zwei Sauerstoffatome nicht direkt benachbart sind" umfasst im Rahmen der vorliegenden Erfindung geradkettige oder verzweigte, gegebenenfalls chirale Alkyl, Alkoxy, Alkenyl, Alkenyloxy, Alkoxyalkyl, Alkenyloxyalkyl und Alkyloxyalkenyl Reste mit 1 bzw. 2 bis 12 Kohlenstoffatomen, welche einfach oder mehrfach mit Fluor substituiert sein können. Bevorzugte Reste sind unsubstituierte, geradkettige Reste mit 1 bzw. 2 bis 6 Kohlenstoffatomen. Bevorzugte Alkenylreste sind diejenigen, worin die Doppelbindung E-konfiguriert ist und sich an C(3) oder, falls Ring A¹ einen gesättigten Ring darstellt, auch an C(1) befindet oder aber endständig ist. Bevorzugte Alkenyloxyreste sind diejenigen mit einer E-konfigurierten Doppelbindung an C(2) oder solche mit einer endständigen Doppelbindung. Beispiele solcher Reste sind Methyl, Aethyl, Propyl, Butyl, Pentyl, Hexyl, Methoxy, Aethoxy, Propyloxy, Butyloxy, Pentyloxy, Hexyloxy, Vinyl, 1E-Propenyl, 1E-Butenyl, 1E-Pentenyl, 1E-Hexenyl, 3-Butenyl, 3E-Pentenyl, 3E-Hexenyl, 4-Pentenyl, 5-Hexenyl, Allyloxy, 2E-Butenyloxy, 3-Butenyloxy, Methoxymethyl, Aethoxymethyl, Propyloxymethyl, Allyloxymethyl, Methoxyäthyl, Aethoxyäthyl, Propyloxyäthyl, Methoxypropyl, Aethoxypropyl, Methoxy-1E-propenyl, Aethoxy-1E-propenyl und dergleichen.

Der Ausdruck "gegebenenfalls mit Fluor substituiertes 1,4-Phenylen" umfasst im Zusammenhang mit Ring A unsubstituierte, mono- oder difluorierte 1,4-Phenylen Ringe.

Das Brückenglied Z bedeutet bevorzugt eine einfache Kovalenzbindung, -CH₂CH₂-, -OCH₂- oder -CH₂O-, besonders bevorzugt jedoch eine einfache Kovalenzbindung oder -CH₂CH₂-.

In den Verbindungen der allgemeinen Formel I, worin X Alkyl, Alkoxy oder Alkoxyalkyl bedeutet, werden geradkettige Reste mit 1 bzw. 2 bis 6 Kohlenstoffatomen bevorzugt, insbesondere werden Reste mit 1 bzw. 2 bis 3 Kohlenstoffatomen bevorzugt, wie beispielsweise Methyl, Aethyl, Propyl, Methoxy, Aethoxy, Propyloxy, Methoxymethyl, Aethoxymethyl, Methoxyethyl und dergleichen.

In den Verbindungen der allgemeinen Formel I, worin X Alkenyl oder Alkenyloxy bedeutet, werden geradkettige Reste mit 2 bis 6 Kohlenstoffatomen bevorzugt. Bevorzugte Alkenyl Reste sind diejenigen, worin die Doppelbindung endständig ist oder E-konfiguriert ist und sich an C(3), oder, an einem trans-1,4-Cyclohexylen Ring, auch an C(1) befindet. Bevorzugte Alkenyloxy Reste sind diejenigen mit einer E-konfigurierten Doppelbindung an C(2) oder solche mit einer endständigen Doppelbindung. Beispiele solcher Reste sind Vinyl, 1E-Propenyl, 1E-Butenyl, 1E-Pentenyl, 1E-Hexenyl, 3-Butenyl, 3E-Pentenyl, 3E-Hexenyl, 4-Pentenyl, 5-Hexenyl, Allyloxy, 2E-Butenyloxy, 3-Butenyloxy und dergleichen.

Besonders bevorzugte Untergruppen der Verbindungen der Formel I sind Verbindungen der allgemeinen Formeln 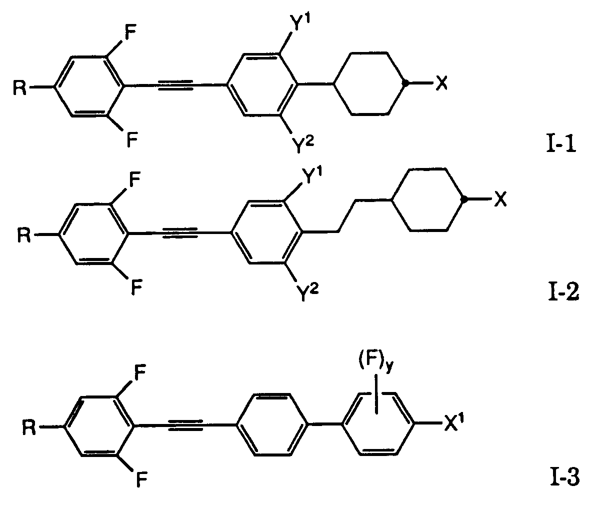 worin
- R: geradkettiges Alkyl, Alkoxy, Alkenyl oder Alkenyloxy mit 1 bzw. 2 bis 6 Kohlenstoffatomen bedeutet, in welchen eine CH₂-Gruppe durch Sauerstoff ersetzt sein kann, mit der Massgabe, dass zwei Sauerstoffatome nicht direkt benachbart sind;
- Y^{1,} Y²: unabhängig voneinander Fluor oder Wasserstoff bedeuten;
- X: Alkyl, Alkenyl, Alkoxy oder Alkenyloxy mit 1 bzw. 2 bis 3 Kohlenstoffatomen, -CH=CF₂, -CH=CHCl, -CH=CHF bedeutet;
- X¹: Fluor, Chlor, -OCF₃, -OCHF₂, -CH=CF₂, Alkyl, Alkenyl, Alkoxy oder Alkenyloxy mit 1 bzw. 2 bis 3 Kohlenstoffatomen bedeutet;
- y: 0, 1 oder 2 bedeutet; dh. der Phenylring 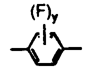
unsubstituiert oder wie folgt substituiert ist

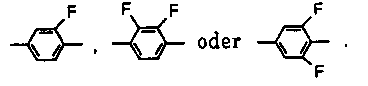

Die gemäss Formel I und I-1 und I-2 definierten Substituenten Y¹ und Y² können voneinander unabhängig sein. Bevorzugt sind diejenigen Verbindungen, bei denen einer dieser Substituenten Wasserstoff und der andere Fluor oder Wasserstoff bedeutet. Ganz besonders bevorzugt sind jedoch diejenigen Verbindungen, bei denen sowohl Y¹ als auch Y² Wasserstoff bedeuten.

Ganz besonders bevorzugte Verbindungen der Formel I-3 sind diejenigen, worin X¹ Fluor, Chlor oder Alkyl mit 1 bis 3 Kohlenstoffatomen bedeutet.

Weitere, ganz besonders bevorzugte Verbindungen der Formel I sind Verbindungen der Formeln I-1 und I-2, worin X Alkyl oder Alkenyl mit 1 bzw. 2 bis 3 Kohlenstoffatomen bedeutet, wie beispielsweise Methyl, Aethyl, Propyl, Vinyl, 1E-Propenyl, oder aber -CH=CF₂, -CH=CHCl oder -CH=CHF darstellt.

Die Herstellung der Verbindungen der Formel I kann in an sich bekannter Weise erfolgen. Sie wird im Schema 1 dargelegt, wobei den in den Formeln I-III angegebenen Substituenten die obenerwähnte Bedeutung zukommt. Die Halogenierung mit Brom oder Iod einer Verbindung IIa zu Verbindungen der Formel III wird vorzugsweise in einem inerten Lösungsmittel (z.B. Tetrahydrofuran, Aether, Dimethoxyäthan usw.) bei tiefer Temperatur (z.B. -70° bis -40°C) durchgeführt.

Metallkatalysierte Kupplungen von Phenylverbindungen mit Acetylenen, wie sie im untenstehenden Schema zur Herstellung von I aus III dargestellt sind, sind grundsätzlich aus der Literatur bekannt, z.B. *C. Pogh and V. Percey, Mol. Cryst. Liq. Cryst.* (1990) 178, 193. Sie können wie bereits erwähnt mit Bromiden oder Iodiden, in einigen Fällen aber auch mit Trifluorsulfonaten oder Chloriden durchgeführt werden. Dabei wird zunächst eine Verbindung der Formel III z.B. mit 2-Methyl-3-butin-2-ol in einem inerten Lösungsmittel wie beispielsweise Tetrahydrofuran mit katalytischen Mengen von Bis-triphenylphosphinpalladiumchlorid, Kupfer-I-iodid und Triphenyl-phosphin in Anwesenheit von Triethylamin bei erhöhter Temperatur (vorzugsweise Rückflusstemperatur des Reaktionsgemisches) umgesetzt. Danach werden Kaliumhydroxid und Tetrabutylammonium-hydrogensulfat, sowie die Verbindung des Typs IIb, zugefügt und bis zum vollständigen Umsatz bei weiterhin erhöhter Temperatur reagieren gelassen. Bei den Verbindungen des Typs IIb können anstelle von Bromiden grundsätzlich auch analoge Iodide, Trifluormethylsulfonate und homologe Polyfluoralkylsulfonate eingesetzt werden. Ueblicherweise wird dabei das einfach gekuppelte Acetylen (IIIa) isoliert, die Schutzgruppe, hier Isopropyloxy, abgespalten und in einer nächsten Stufe mit einer Verbindung der allgemeinen Formel IIb gekuppelt. Der Umsatz von Zwischenprodukten der Formel III zu Verbindungen der Formel I kann aber auch in einer Stufe, d.h. ohne dazwischenliegende Isolierung der Verbindung der Formel IIIa, realisiert werden kann.

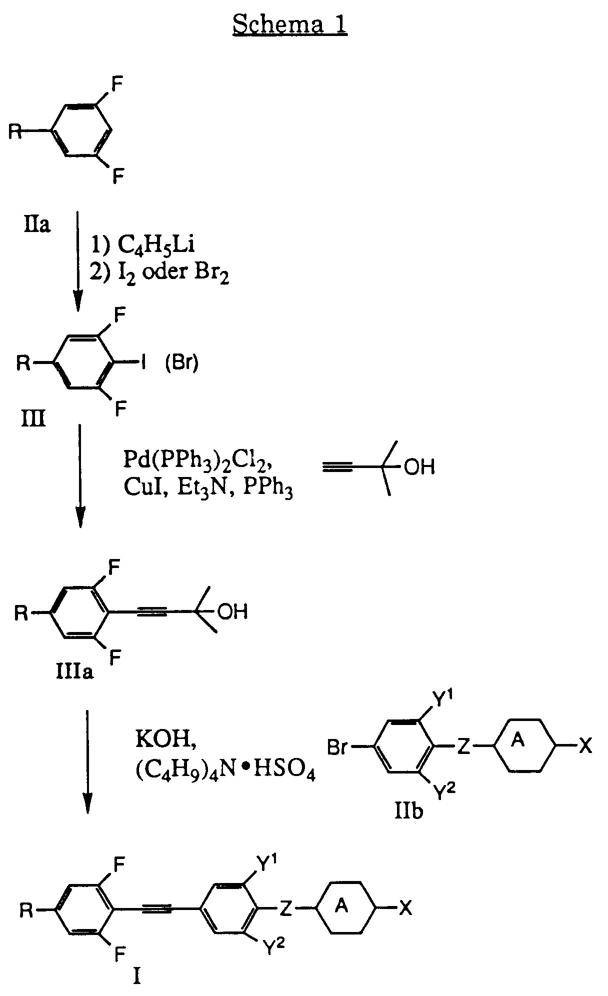

Die Ausgangsmaterialien der Formel IIa sind bekannte oder analoge bekannter Verbindungen. Sie können nach an sich bekannten Methoden, beispielsweise aus kommerziell erhältlichem 3,5-Difluorbenzaldehyd durch Wittigreaktion und anschliessende Hydrierung, oder durch Verätherung von ebenfalls kommerziellem 3,5-Difluorphenol hergestellt werden. Geeignet substituierte Phenylderivate der Formel IIb sind vielfach im Handel erhältlich oder können leicht aus känflichen Vorstufen nach dem Fachmann bekannten Methoden modifiziert werden.

Die erfindungsgemässen flüssigkristallinen Gemische enthalten mindestens zwei Komponenten, wovon mindestens eine Komponente eine Verbindung der Formel I ist. Eine zweite, und gegebenenfalls weitere Komponenten können weitere Verbindungen der Formel I und/oder andere Flüssigkristallkomponenten sein.

Die Verbindungen der Formel I eignen sich insbesondere für nematische oder, sofern mindestens eine Komponente des Gemisches optisch aktiv ist, auch für cholesterische Gemische. Ein bevorzugter Anwendungsbereich betrifft die Verwendung als Dielektrikum in Flüssigkristallanzeigevorrichtungen mit verdrillt nematischer Flüssigkristallstruktur, wie TN-Zellen, STN-Zellen und TFT-Zellen. Bevorzugte Gemische sind daher diejenigen, welche zusätzlich eine oder mehrere Verbindungen mit positiver dielektrischer Anisotropie enthalten.

Aufgrund der guten Löslichkeit der Verbindungen der Formel I in anderen Flüssigkristallmaterialien und aufgrund ihrer guten Mischbarkeit untereinander kann der Anteil der Verbindungen der Formel I in den erfindungsgemässen Gemischen relativ hoch sein und beispielsweise etwa 1-70 Gew.-% betragen. Im allgemeinen ist ein Anteil von etwa 3-40 Gew.-%, insbesondere 5-30 Gew.-% an Verbindungen der Formel I bevorzugt.

Vorzugsweise enthalten die erfindungsgemässen Gemische neben einer oder mehreren Verbindungen der Formel I, eine oder mehrere Verbindungen aus der Gruppe der Verbindungen der allgemeinen Formeln 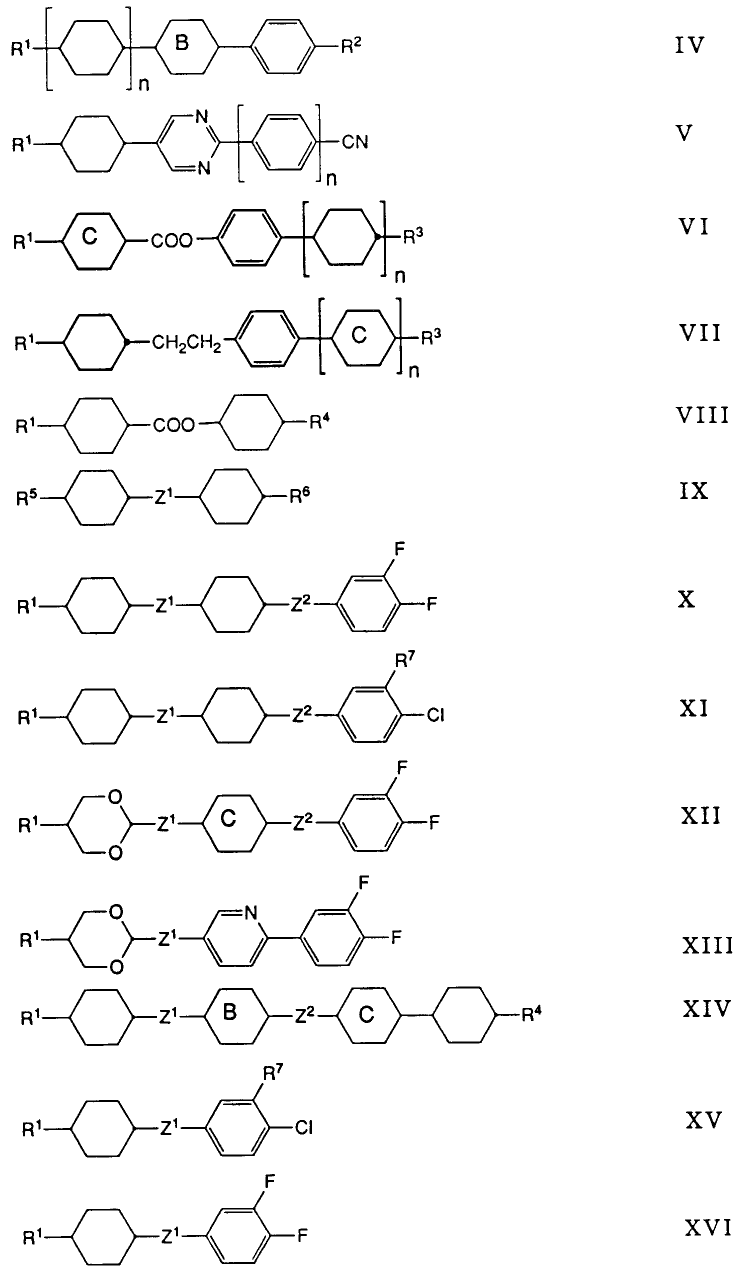 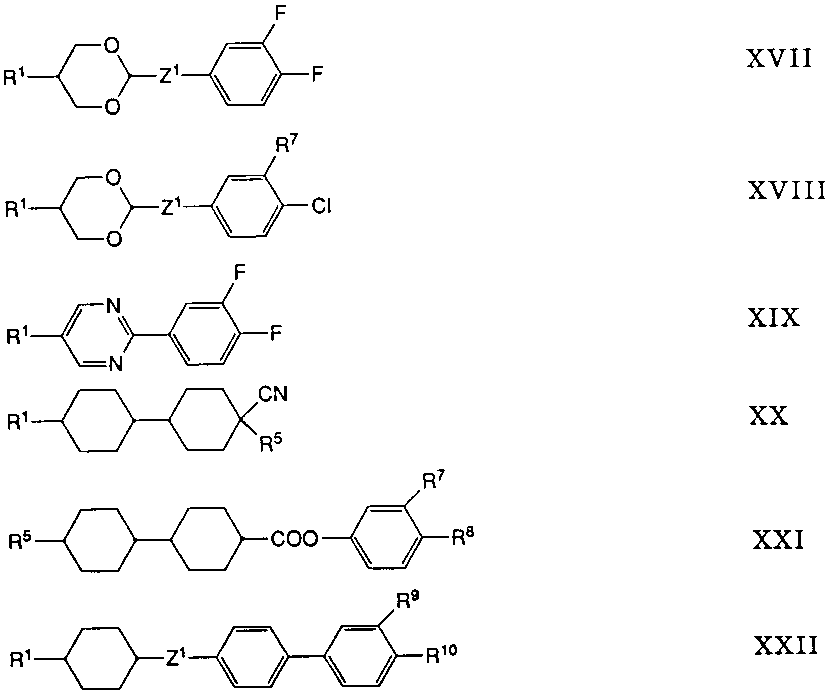 worin
- R¹,R⁴: Alkyl, Alkoxyalkyl, 3E-Alkenyl, 4-Alkenyl oder an gesättigten Ringen auch 1E-Alkenyl bedeuten;
- n: 0 oder 1 bedeutet;
- Ring B: 1,4-Phenylen, Pyridin-2,5-diyl, Pyrimidin-2,5-diyl, trans-1,4-Cyclohexylen oder trans-1,3-Dioxan-2,5-diyl bezeichnet;
- R²: Cyano, Isothiocyanato, Fluor, Alkyl, 3E-Alkenyl, 4-Alkenyl, Alkoxy, 2E-Alkenyloxy, 3-Alkenyloxy oder 1-Alkinyl darstellt;
- Ring C: 1,4-Phenylen oder trans-1,4-Cyclohexylen bedeutet;
- R³: Alkyl, 3E-Alkenyl, 4-Alkenyl, oder an trans-1,4-Cyclohexylen auch 1E-Alkenyl, oder an 1,4-Phenylen auch Cyano, Isothiocyanato, Alkoxy, 2E-Alkenyloxy oder 3-Alkenyloxy bezeichnet;
- R⁵: Alkyl, 1E-Alkenyl, 3E-Alkenyl oder 4-Alkenyl bedeutet;
- R⁶: Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl darstellt;
- Z¹, Z²: unabhängig voneinander eine einfache Kovalenzbindung oder -CH₂CH₂- bezeichnen, wobei zwei aromatische Ringe immer durch eine einfache Kovalenzbindung verknüpft sind;
- R⁷: Wasserstoff, Fluor oder Chlor bedeutet;
- R⁸: Cyano, Fluor oder Chlor darstellt;
- R⁹: Wasserstoff oder Fluor bezeichnet; und
- R¹⁰: Fluor oder Chlor darstellt.

Der obige Ausdruck "gesättigter Ring" umfasst trans-1,4-Cyclohexylen und trans-1,3-Dioxan-2,5-diyl. Die Reste R¹ bis R⁶ besitzen vorzugsweise je 1 bis 12 Kohlenstoffatome, besonders bevorzugt je 1 bis 7 Kohlenstoffatome. Geradkettige Reste sind im allgemeinen bevorzugt.

Der Ausdruck "Alkyl" bedeutet in diesem Zusammenhang vorzugsweise geradkettige Reste mit 1 bis 12 Kohlenstoffatomen, vorzugsweise mit 1 bis 7 Kohlenstoffatomen, wie beispielsweise Methyl, Aethyl, Propyl, Butyl, Pentyl, Hexyl oder Heptyl und dergleichen.

Der Ausdruck "Alkyloxyalkyl" bedeutet in diesem Zusammenhang vorzugsweise geradkettige Reste mit 1 bis 12 Kohlenstoffatomen, besonders bevorzugt mit 1 bis 7 Kohlenstoffatomen, wie beispielsweise Methoxymethyl, Aethoxymethyl, Propyloxymethyl, Butyloxymethyl, Methoxypropyl und dergleichen.

Der Ausdruck "Alkyloxy" bedeutet in diesem Zusammenhang vorzugsweise geradkettige Reste mit 1 bis 12 Kohlenstoffatomen, besonders bevorzugt mit 1 bis 7 Kohlenstoffatomen, wie beispielsweise Methoxy, Aethoxy, Propyloxy, Butyloxy, Pentyloxy, Hexyloxy, Heptyloxy und dergleichen.

Der Ausdruck "1E-Alkenyl" bedeutet in diesem Zusammenhang vorzugsweise geradkettige Alkenylreste mit 2 bis 12, besonders bevorzugt mit 2 bis 7 Kohlenstoffatomen, in denen die Doppelbindung sich in 1-Stellung befindet, wie beispielsweise Vinyl, 1E-Propenyl, 1E-Butenyl, 1E-Pentenyl, 1E-Hexenyl, 1E-Heptenyl und dergleichen.

Der Ausdruck "3E-Alkenyl" bedeutet in diesem Zusammenhang vorzugsweise geradkettige Alkenylreste mit 4 bis 12, besonders bevorzugt mit 4 bis 7 Kohlenstoffatomen, in denen die Doppelbindung sich in 3-Stellung befindet, wie beispielsweise 3-Butenyl, 3E-Pentenyl, 3E-Hexenyl, 3E-Heptenyl und dergleichen.

Der Ausdruck "4-Alkenyl" bedeutet in diesem Zusammenhang vorzugsweise geradkettige Alkenylreste mit 5 bis 12 Kohlenstoffatomen, in denen die Doppelbindung sich in 4-Stellung befindet, wie beispielsweise 4-Pentenyl, 4-Hexenyl, 4-Heptenyl und dergleichen.

Der Ausdruck "2E- bzw. 3Z-Alkenyloxy" bedeutet in diesem Zusammenhang vorzugsweise geradkettige Alkenyloxyreste mit 3 bzw. 4 bis 12 Kohlenstoffatomen, besonders bevorzugt mit 3 bzw. 4 bis 7 Kohlenstoffatomen, in denen die Doppelbindung sich in 2- bzw. 3-Stellung befindet und E bzw. Z die bevorzugte Konfiguration angibt, wie beispielsweise Allyloxy, 2E-Butenyloxy, 2E-Pentenyloxy, 2E-Hexenyloxy, 2E-Heptenyloxy, 3-Butenyloxy, 3Z-Pentenyloxy, 3Z-Hexenyloxy, 3Z-Heptenyloxy, 4-Pentenyloxy, 5-Hexenyloxy, 6-Heptenyloxy und dergleichen.

Der Ausdruck "1-Alkinyl" bedeutet in diesem Zusammenhang vorzugsweise geradkettige Alkinylreste mit 2 bis 12, besonders bevorzugt mit 2 bis 7 Kohlenstoffatomen, in denen die Dreifachbindung sich in 1-Stellung befindet, wie beispielsweise Aethinyl, 1-Propinyl, 1-Butinyl, 1-Pentinyl und dergleichen.

Die Herstellung der flüssigkristallinen Gemische und der elektrooptischen Vorrichtungen kann in an sich bekannter Weise erfolgen.

Die Erfindung wird durch die folgenden Beispiele weiter veranschaulicht. In den Beispielen bedeutet C eine kristalline, N eine nematische, S eine smektische und I die isotrope Phase. V₁₀ bezeichnet die Spannung für 10% Transmission. tₒₙ und t_{off} bezeichnen die Einschaltzeit bzw. die Ausschaltzeit. Δn bezeichnet die optische Anisotropie.

### Beispiel 1

a) Zu einer Lösung von 5 g 1-Propyl-3,5-difluorbenzol in 50 ml trockenem Tetrahydrofuran bei -70°C wurden 22 ml einer 1,6 N Butyllithiumlösung in Hexan während 30 Min. getropft und während 1 Std. bei -70°C reagieren gelassen. Dann wurde bei -60° eine Lösung von 8,95 g Iod in 20 ml trockenem Tetrahydrofuran innert 10 Min. zugetropft und während weiteren 30 Min. allmählich auf Raumtemperatur erwärmt. Die entstandene gelbe Lösung wurde danach mit 40 ml einer 10proz. wässrigen Natriumbicarbonatlösung versetzt und mit Ether extrahiert. Die Etherlösung wurde mit ges. Kochsalzlösung und mehrmals mit Wasser gewaschen, über Magnesiumsulfat getrocknet, filtriert und eingeengt. Chromatographie des Rückstandes über 200 g Kieselgel mit Hexan ergab 1-Propyl-3,5-difluor-4-iodbenzol als farblose Flüssigkeit.
b) Ein Gemisch von 2 g 1-Propyl-3,5-difluor-4-iodbenzol, 0,626 g 2-Methyl-3-butin-2-ol, 0,164 g Tetrakis(triphenylphosphin)palladium(0), 10 ml Triethylamin und 0,054 g Kupfer(I)iodid wird während 2 Std. bei 90° gerührt. Dann wurde das abgekühlte Reaktionsgemisch zwischen Wasser und Ether verteilt, die Etherphase mehrmals mit Wasser gewaschen, über Magnesiumsulfat getrocknet, über Celite filtriert und am Ratationsverdampfer eingedampft. Der Rückstand wurde hierauf an 75 g Kieselgel (Laufmittel: 10% Essigester in Hexan) chromatographiert und erneut eingedampft. Es entstanden 1,5 g 4-(4-Propyl-2,6-difluorphenyl)-2-methylbutin-2-ol als gelbe Flüssigkeit.
c) Ein Gemisch von 0,551 g 4-(4-Propyl-2,6-difluorphenyl)-2-methylbutin-2-ol, 0,68 g 1-Brom-4-(4-trans-ethylcyclohexyl)benzol, 195 g Kaliumhydroxyd, 0,054 g Tetrakis(triphenylphosphin)palladium(0) und 5 ml Triethylamin wurden während 16 Std. unter Rückfluss reagieren gelassen. Dann wurde das abgekühlte Reaktionsgemisch zwischen Wasser und Ether verteilt, die Etherphase mehrmals mit Wasser gewaschen, über Magnesiumsulfat getrocknet ,über Celite filtriert und am Rotationsverdampfer eingedampft. Der Rückstand wurde hierauf an 100 g Kieselgel mit Hexan chromatographiert. Zweimalige Kristallisation aus Hexan ergab 0,37 g 2,6-Difluor-4-propyl-4'-(4-trans-ethylcyclohexyl)tolan. Smp. 55,6°C, Klp. (N/I) 161,8°C.

Auf analoge Weise können die folgenden Verbindungen hergestellt werden:
2,6-Difluor-4-ethyl-4'-(4-ethylphenyl)tolan;
2,6-Difluor-4-propyl-4'-(4-ethylphenyl)tolan;
2,6-Difluor-4-propyl-3'-fluor-4'-(4-ethylphenyl)tolan;
2,6-Difluor-4-butyl-4'-(4-ethylphenyl)tolan;
2,6-Difluor-4-(3-butenyl)-4'-(4-ethylphenyl)tolan;
2,6-Difluor-4-pentyl-4'-(4-ethylphenyl)tolan;
2,6-Difluor-4-(4-pentenyl)-4'-(4-ethylphenyl)tolan;
2,6-Difluor-4-propyloxy-4'-(4-ethylphenyl)tolan;
2,6-Difluor-4-(3-butenyloxy)-4'-(4-ethylphenyl)tolan;
2,6-Difluor-4-ethyl-4'-(4-propylphenyl)tolan;
2,6-Difluor-4-propyl-4'-(4-propylphenyl)tolan;
2,6-Difluor-4-pentyl-4'-(4-propylphenyl)tolan;
2,6-Difluor-4-propyl-4'-(4-methoxyphenyl)tolan;
2,6-Difluor-4-butyl-4'-(4-ethoxyphenyl)tolan;
2,6-Difluor-4-propyl-4'-(3-fluor-4-ethylphenyl)tolan;
2,6-Difluor-4-propyl-4'-(3-fluorphenyl)tolan;
2,6-Difluor-4-propyl-4'-(3-chlorphenyl)tolan;
2,6-Difluor-4-propyl-4'-(3,4-difluorophenyl)tolan;
2,6-Difluor-4-butyl-4'-(3,4-difluorophenyl)tolan;
2,6-Difluor-4-pentyl-4'-(3,4-difluorophenyl)tolan;
2,6-Difluor-4-(4-pentenyl)-4'-(3,4-difluorophenyl)tolan;
2,6-Difluor-4-propyl-4'-(4-trifluoromethylphenyl)tolan;
2,6-Difluor-4-propyl-4'-(3-fluor-4-trifluoromethylphenyl)tolan;
2,6-Difluor-4-propyl-4'-(3-fluor-4-chlorphenyl)tolan, Smp. (C/N)
70,7°C, Klp. (N/I) 165,9°C;
2,6-Difluor-4-propyl-4'-(4-difluoromethoxyphenyl)tolan;
2,6-Difluor-4-propyl-4'-(3-fluor-4-difluoromethoxyphenyl)tolan;
2,6-Difluor-4-propyl-4'-(4-trifluoromethoxyphenyl)tolan;
2,6-Difluor-4-propyl-4'-[4-(2,2-difluorovinyl)phenyl]tolan;
2,6-Difluor-4-propyl-4'-[4-(2-fluorovinyl)phenyl]tolan;
2,6-Difluor-4-propyl-3'-fluor-4'-[4-(2,2-difluorovinyl)phenyl]tolan;
2,6-Difluor-4-propyl-3'-fluor-4'-[4-(2-fluorovinyl)phenyl]tolan;
2,6-Difluor-4-butyl-4'-(4-trans-ethylcyclohexyl)tolan;
2,6-Difluor-4-pentyl-4'-(4-trans-ethylcyclohexyl)tolan;
2,6-Difluor-4-propyloxy-4'-(4-trans-ethylcyclohexyl)tolan;
2,6-Difluor-4-propyl-4'-(4-trans-vinylcyclohexyl)tolan, Smp. (C/N)
56,3°C, Klp (N/I) 184,9°C;
2,6-Difluor-4-propyl-4'-[4-trans-(2,2-difluorovinyl)cyclohexyl]tolan;
2,6-Difluor-4-propyl-4'-[4-trans-(2-fluorovinyl)cyclohexyl]tolan;
2,6-Difluor-4-propyl-4'-[4-trans-(2-E-chlorovinyl)cyclohexyl]tolan;
2,6-Difluor-4-propyl-4'-(4-trans-propylcyclohexyl)tolan;
2,6-Difluor-4-propyl-4'-[4-trans-(1-E-propenyl)cyclohexyl]tolan;
2,6-Difluor-4-propyl-4'-(4-trans-butylcyclohexyl)tolan;
2,6-Difluor-4-propyl-4'-[4-trans-(3-butenyl)cyclohexyl]tolan;
2,6-Difluor-4-butyl-4'-(4-trans-propylcyclohexyl)tolan;
2,6-Difluor-4-pentyl-4'-(4-trans-propylcyclohexyl)tolan;
2,6-Difluor-4-allyloxy-4'-(4-trans-propylcyclohexyl)tolan;
2,6-Difluor-4-propyl-4'-(4-trans-methoxycyclohexyl)tolan;
2,6-Difluor-4-propyl-4'-(4-trans-ethoxycyclohexyl)tolan;
2,6-Difluor-4-butyl-4'-(4-trans-ethoxycyclohexyl)tolan;
2,6-Difluor-4-pentyl-4'-(4-trans-methoxycyclohexyl)tolan;
2,6-Difluor-4-propyl-4'-(4-trans-allyloxycyclohexyl)tolan;
2,6-Difluor-4-propyl-4'-(4-trans-methoxymethylcyclohexyl)tolan;
2,6-Difluor-4-butyl-4'-(4-trans-methoxymethylcyclohexyl)tolan;
2,6-Difluor-4-pentyl-4'-(4-trans-methoxymethylcyclohexyl)tolan;
2,6-Difluor-4-propyl-4'-[2-(4-trans-ethylcyclohexyl)ethyl]tolan;
2,6-Difluor-4-butyl-4'-[2-(4-trans-ethylcyclohexyl)ethyl]tolan;
2,6-Difluor-4-pentyl-4'-[2-(4-trans-ethylcyclohexyl)ethyl]tolan;
2,6-Difluor-4-propyl-4'-[2-(4-trans-vinylcyclohexyl)ethyl]tolan;
2,6-Difluor-4-propyl-4'-{2-[4-trans-(2,2-difluorvinyl)cyclohexyl]ethyl}tolan;
2,6-Difluor-4-propyl-4'-{2-[4-trans-(2-fluorvinyl)cyclohexyl]ethyl}tolan;
2,6-Difluor-4-butyl-4'-{2-[4-trans-(2,2-difluorvinyl)cyclohexyl]ethyl}tolan;
2,6-Difluor-4-butyl-4'-{2-[4-trans-(2-fluorvinyl)cyclohexyl]ethyl}tolan;
2,6-Difluor-4-pentyl-4'-{2-[4-trans-(2,2-difluorvinyl)cyclohexyl]ethyl}tolan;
2,6-Difluor-4-pentyl-4'-{2-[4-trans-(2-fluorvinyl)cyclohexyl]ethyl}tolan;
2,6-Difluor-4-propyl-4'-{2-[4-trans-(2-E-chlorvinyl)cyclohexyl]ethyl}tolan;
2,6-Difluor-4-propyl-4'-[2-(4-trans-methoxycyclohexyl)ethyl]tolan;
2,6-Difluor-4-butyl-4'-[2-(4-trans-methoxycyclohexyl)ethyl]tolan;
2,6-Difluor-4-pentyl-4'-[2-(4-trans-methoxycyclohexyl)ethyl]tolan;
2,6-Difluor-4-propyl-4'-[2-(4-trans-ethoxycyclohexyl)ethyl]tolan;
2,6-Difluor-4-propyl-4'-[2-(4-trans-methoxymethylcyclohexyl)ethyl]tolan;

### Beispiel 2

Zur Untersuchung der Eigenschaften der Verbindungen der Formel I in Gemischen wurden binäre Mischungen (BM) mit 4-(trans-4-Pentylcyclohexyl)benzonitril hergestellt. Die Schwellenspannung und die Ansprechzeiten wurden in einer TN-Zelle (low bias tilt) mit 8 mm Plattenabstand bei 22°C gemessen; als Betriebsspannung wurde der 2,5-fache Wert der Schwellenspannung (V₁₀) gewählt. Die entsprechenden Daten für 4-(trans-4-Pentylcyclohexyl)benzonitril betragen: Klp. (N/I) = 54.6°C, V₁₀ = 1,62 V, tₒₙ = 22 ms, t_{off} = 42 ms, Δn = 0,120.

### BM-1

- 90 Gew.%: 4-(trans-4-Pentylcyclohexyl)benzonitril
- 10 Gew.%: 2,6-Difluor-4-propyl-4'-(3-fluor-4-chlorphenyl)tolan;
- Klp. (N/I):: 59°C, V₁₀ = 1,54 V, tₒₙ = 28 ms, t_{off} = 46 ms, Δn = 0,143

### BM-2

- 80 Gew.%: 4-(trans-4-Pentylcyclohexyl)benzonitril
- 20 Gew.%: 2,6-Difluor-4-propyl-4'-(3-fluor-4-chlorphenyl)tolan;
- Klp. (N/I):: 64,9°C, V₁₀ = 1,54 V, tₒₙ = 29 ms, t_{off} = 48 ms, Δn = 0,155

### BM-3

- 90 Gew.%: 4-(trans-4-Pentylcyclohexyl)benzonitril
- 10 Gew.%: 2,6-Difluor-4-propyl-4'-(4-trans-ethylcyclohexyl)tolan;
- Klp. (N/I):: 59,6°C, V₁₀ = 1,52V, tₒₙ = 27 ms, t_{off} = 42 ms, Δn = 0,133

### BM-4

- 80 Gew.%: 4-(trans-4-Pentylcyclohexyl)benzonitril
- 20 Gew.%: 2,6-Difluor-4-propyl-4'-(4-trans-ethylcyclohexyl)tolan;
- Klp. (N/I):: 66,5°C, V₁₀ = 1,58 V, tₒₙ = 28 ms, t_{off} = 43 ms, Δn = 0,144

### BM-5

- 90 Gew.%: 4-(trans-4-Pentylcyclohexyl)benzonitril
- 10 Gew.%: 2,6-Difluor-4-propyl-4'-(4-trans-vinylcyclohexyl)tolan;
- Klp. (N/I):: 61,6°C, V₁₀ = 1,50V, tₒₙ = 25 ms, t_{off} = 40 ms, Δn = 0,135

### BM-6

- 80 Gew.%: 4-(trans-4-Pentylcyclohexyl)benzonitril
- 20 Gew.%: 2,6-Difluor-4-propyl-4'-(4-trans-vinylcyclohexyl)tolan;
- Klp. (N/I):: 70,1°C, V₁₀ = 1,70 V, tₒₙ = 26 ms, t_{off} = 42 ms, Δn = 0,148

## Patentansprüche

1. Verbindungen der allgemeinen Formel 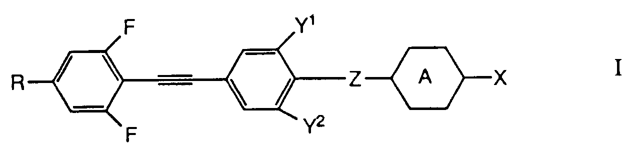 worin
R Alkyl, Alkoxy, Alkenyl oder Alkenyloxy mit 1 bzw. 2 bis 12 Kohlenstoffatomen bedeutet, in welchen eine CH₂-Gruppe durch Sauerstoff und/oder eines oder mehrere Wasserstoffatome durch Fluoratome ersetzt sein können, mit der Massgabe, dass zwei Sauerstoffatome nicht direkt benachbart sind;
Y^{1,} Y² unabhängig voneinander Fluor oder Wasserstoff bedeuten,
Z eine einfache Kovalenzbindung, -CH₂CH₂-, -OCH₂-, -CH₂O-, -(CH₂)₄-, -O(CH₂)₃-, -(CH₂)₃O-, oder, falls Ring A einen gesättigten Ring darstellt, auch die trans-Form von -(CH₂)₂CH=CH- oder -CH₂OCH=CH- bedeutet;
A trans-1,4-Cyclohexylen oder, gegebenenfalls mit Fluor substituiertes, 1,4-Phenylen bedeutet;
X Alkyl, Alkenyl, Alkoxy, Alkenyloxy oder Alkoxyalkyl mit 1 bzw. 2 bis 6 Kohlenstoffatomen, -CH=CF₂, oder an trans-1,4-Cyclohexylen auch -CH=CHCl oder -CH=CHF, oder an 1,4-Phenylen auch Fluor, Chlor, -CF₃, -OCF₃, oder -OCHF₂ bedeutet.

2. Verbindungen gemäss Anspruch 1, dadurch gekennzeichnet, dass Z eine einfache Kovalenzbindung, -CH₂CH₂-, -OCH₂- oder -CH₂O-, bedeutet.

3. Verbindungen gemäss einem der Ansprüche 1 oder 2, der Formeln 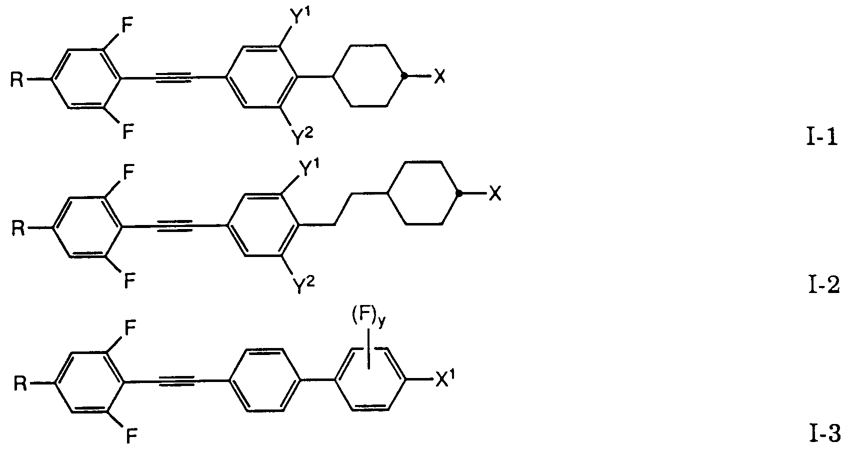 worin
R geradkettiges Alkyl, Alkoxy, Alkenyl oder Alkenyloxy mit 1 bzw. 2 bis 6 Kohlenstoffatomen bedeutet, in welchen eine CH₂-Gruppe durch Sauerstoff ersetzt sein kann, mit der Massgabe, dass zwei Sauerstoffatome nicht direkt benachbart sind;
Y^{1,} Y² unabhängig voneinander Fluor oder Wasserstoff bedeuten;
X Alkyl, Alkenyl, Alkoxy oder Alkenyloxy mit 1 bzw. 2 bis 3 Kohlenstoffatomen, -CH=CF₂ oder -CH=CHCl oder -CH=CHF bedeutet;
X¹ Fluor, Chlor, -CF₃, -OCF₃, -OCHF₂, -CH=CF₂, Alkyl, Alkenyl, Alkoxy oder Alkenyloxy mit 1 bzw. 2 bis 3 Kohlenstoffatomen bedeutet;
y 0, 1 oder 2 bedeutet; dh. der Phenylring 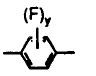
unsubstituiert oder wie folgt substituiert ist

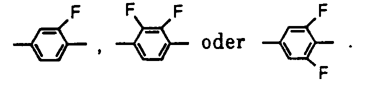

4. Verbindungen gemäss einem der Ansprüche 1 bis 3, dadurch gekennzeichnet, dass einer der Substituenten Y¹ oder Y² Wasserstoff und der andere Wasserstoff oder Fluor bedeutet.

5. Verbindungen gemäss Anspruch 4, dadurch gekennzeichnet, dass beide Substituenten Y¹ oder Y² Wasserstoff bedeuten.

6. Verbindungen gemäss einem der Ansprüche 1 bis 5, dadurch gekennzeichnet, dass X Alkyl mit 1 bis 3 Kohlenstoffatomen bedeutet; und X¹ Fluor, Chlor oder Alkyl mit 1 bis 3 Kohlenstoffatomen bedeutet.

7. Verbindungen gemäss einem der Ansprüche 1 bis 5, dadurch gekennzeichnet, dass X Alkyl oder Alkenyl mit 1 bzw. 2 bis 3 Kohlenstoffatomen, -CH=CF₂, -CH=CHCl oder -CH=CHF darstellt.

8. Flüssigkristalline Gemische enthaltend mindestens zwei Komponenten, dadurch gekennzeichnet, dass mindestens eine Komponente eine Verbindung der in Anspruch 1 definierten Formel I ist.

9. Flüssigkristalline Gemische gemäss Anspruch 8, dadurch gekennzeichnet, dass der Anteil an Verbindungen der in Anspruch 1 definierten Formel I 3-40 Gew.-% beträgt.

10. Flüssigkristalline Gemische gemäss einem der Ansprüche 8 oder 9, dadurch gekennzeichnet, dass der Anteil an Verbindungen der in Anspruch 1 definierten Formel I 5-30 Gew.-% beträgt.

11. Verwendung der in Anspruch 1 definierten Verbindungen der Formel I für elektro-optische Zwecke.

12. Verwendung von flussigkristallinen Gemischen gemäss einem der Ansprüche 8 bis 10 für elektro-optische Zwecke.

## Claims

1. Compounds of the general formula 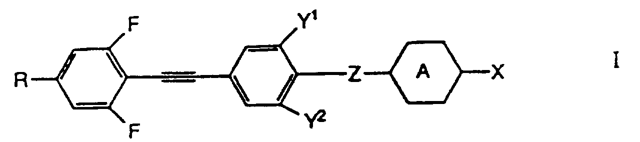 in which
R is alkyl, alkoxy, alkenyl or alkenyloxy having 1 or 2 to 12 carbon atoms, in which one CH₂ group may be replaced by oxygen and/or one or more hydrogen atoms may be replaced by fluorine atoms, with the proviso that two oxygen atoms are not directly adjacent;
Y¹ and Y², independently of one another, are fluorine or hydrogen,
Z is a single covalent bond, -CH₂CH₂-, -OCH₂-, -CH₂O-, -(CH₂)₄-, -O(CH₂)₃-, -(CH₂)₃O- or, if ring A is a saturated ring, Z is alternatively the trans-form of -(CH₂)₂CH=CH- or -CH₂OCH=CH-;
A is trans-1,4-cyclohexylene or optionally fluorine-substituted 1,4-phenylene;
X is alkyl, alkenyl, alkoxy, alkenyloxy or alkoxyalkyl having 1 or 2 to 6 carbon atoms, -CH=CF₂ or, on trans-1,4-cyclohexylene, alternatively -CH=CHCl or -CH=CHF, or, on 1,4-phenylene, alternatively fluorine, chlorine, -CF₃, -OCF₃ or -OCHF₂.

2. Compounds according to Claim 1, characterized in that Z is a single covalent bond, -CH₂CH₂-, -OCH₂- or -CH₂O-.

3. Compounds according to Claim 1 or 2, of the formulae 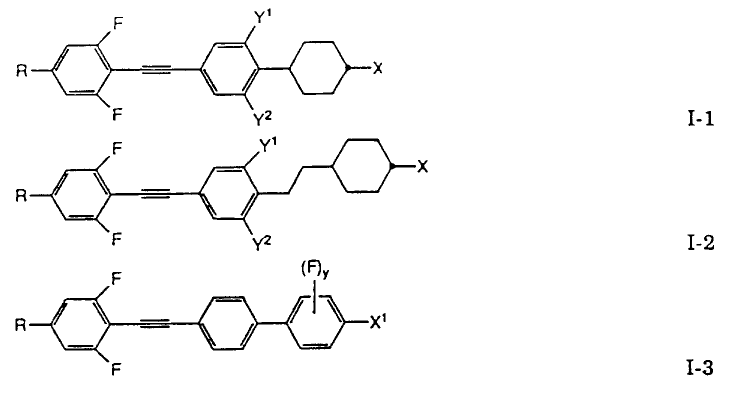 in which
R is straight-chain alkyl, alkoxy, alkenyl or alkenyloxy having 1 or 2 to 6 carbon atoms, in which one CH₂ group may be replaced by oxygen, with the proviso that two oxygen atoms are not directly adjacent;
Y¹ and Y², independently of one another, are fluorine or hydrogen;
X is alkyl, alkenyl, alkoxy or alkenyloxy having 1 or 2 to 3 carbon atoms, -CH=CF₂ or -CH=CHCl or -CH=CHF;
X¹ is fluorine, chlorine, -CF₃, -OCF₃, -OCHF₂, -CH=CF₂, alkyl, alkenyl, alkoxy or alkenyloxy having 1 or 2 to 3 carbon atoms;
y is 0, 1 or 2, i.e. the phenyl ring 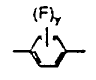
is unsubstituted or substituted as follows

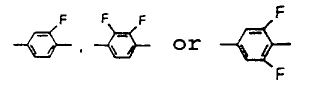

4. Compounds according to one of Claims 1 to 3, characterized in that one of the substituents Y¹ or Y² is hydrogen and the other is hydrogen or fluorine.

5. Compounds according to Claim 4, characterized in that both substituents Y¹ and Y² are hydrogen.

6. Compounds according to one of Claims 1 to 5, characterized in that X is alkyl having 1 to 3 carbon atoms, and X¹ is fluorine, chlorine or alkyl having 1 to 3 carbon atoms.

7. Compounds according to one of Claims 1 to 5, characterized in that X is alkyl or alkenyl having 1 or 2 to 3 carbon atoms, -CH=CF₂, -CH=CHCl or -CH=CHF.

8. Liquid-crystalline mixtures comprising at least two components, characterized in that at least one component is a compound of the formula I defined in Claim 1.

9. Liquid-crystalline mixtures according to Claim 8, characterized in that the proportion of compounds of the formula I defined in Claim 1 is 3-40% by weight.

10. Liquid-crystalline mixtures according to Claim 8 or 9, characterized in that the proportion of compounds of the formula I defined in Claim 1 is 5-30% by weight.

11. Use of the compounds of the formula I defined in Claim 1 for electro-optical purposes.

12. Use of liquid-crystalline mixtures according to one of Claims 8 to 10 for electro-optical purposes.

## Revendications

1. Composés de formule générale 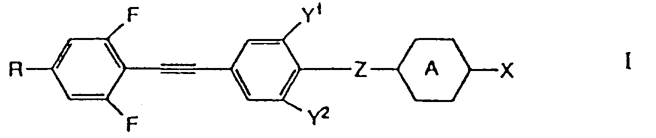 dans laquelle
R représente un groupe alkyle, alcoxy, alcényle ou alcényloxy ayant de 1 ou, respectivement, 2 à 12 atomes de carbone, dans lesquels un groupe CH₂ peut être remplacé par un atome d'oxygène et/ou un ou plusieurs atomes d'hydrogène peuvent être remplacés par des atomes de fluor, étant entendu que 2 atomes d'oxygène ne sont pas immédiatement voisins,
Y¹, Y² représentent, indépendamment l'un de l'autre, un atome de fluor ou d'hydrogène,
Z représente une liaison covalente simple, -CH₂CH₂-, -OCH₂-, -CH₂O-, -(CH₂)₄-, -O(CH₂)₃-, -(CH₂)₃O- ou, lorsque le cycle A représente un cycle saturé, également la forme *trans* de -(CH₂)₂CH=CH- ou de -CH₂OCH=CH-;
A représente le radical *trans*-1,4-cyclohexylène ou un radical 1,4-phénylène éventuellement substitué par le fluor;
X représente un groupe alkyle, alcényle, alcoxy, alcényloxy ou alcoxyalkyle ayant de 1 ou, respectivement, 2 à 6 atomes de carbone, -CH=CF₂ ou, sur le radical *trans*-1,4-cyclohexylène, également -CH=CHCl ou -CH=CHF ou, sur le radical 1,4-phénylène, également un atome de fluor ou de chlore ou le groupe -CF₃, -OCF₃ ou -OCHF₂.

2. Composés selon la revendication 1, caractérisés en ce que Z représente une liaison covalente simple, -CH₂CH₂-, -OCH₂- ou -CH₂O-.

3. Composés selon la revendication 1 ou 2, de formules 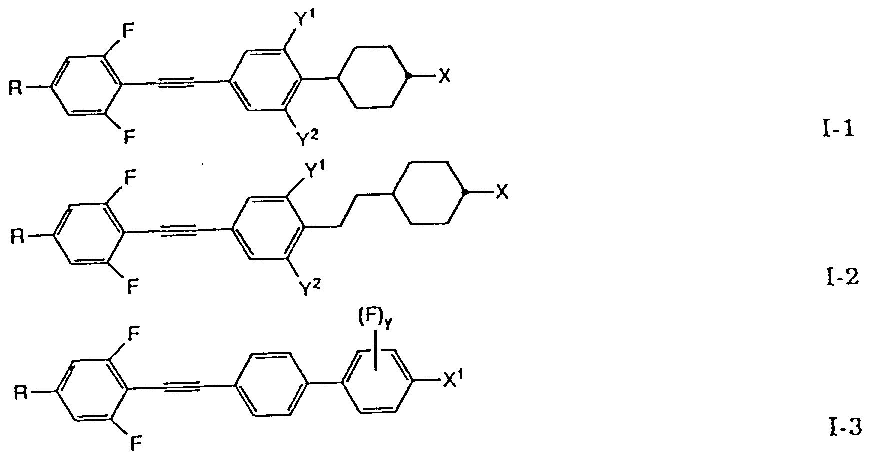 dans lesquelles
R représente un groupe alkyle, alcoxy, alcényle ou alcényloxy à chaîne droite, ayant de 1 ou, respectivement, 2 à 6 atomes de carbone, dans lesquels un groupe CH₂ peut être remplacé par un atome d'oxygène, étant entendu que deux atomes d'oxygène ne sont pas immédiatement voisins;
Y¹, Y² représentent, indépendamment l'un de l'autre, un atome de fluor ou d'hydrogène;
X représente un groupe alkyle, alcényle, alcoxy ou alcényloxy ayant de 1 ou, respectivement, 2 à 3 atomes de carbone, -CH=CF₂ ou -CH=CHCl ou -CH=CHF;
X¹ représente un atome de fluor ou de chlore, ou un groupe -CF₃, -OCF₃, -OCHF₂, -CH=CF₂, alkyle, alcényle, alcoxy ou alcényloxy ayant de 1 ou, respectivement, 2 à 3 atomes de carbone;
y représente 0, 1 ou 2; c'est-à-dire que le noyau phényle 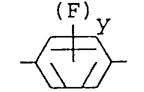
est non substitué ou substitué comme suit

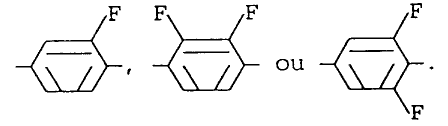

4. Composés selon l'une des revendications 1 à 3, caractérisés en ce que l'un des substituants Y¹ et Y² représente un atome d'hydrogène et l'autre représente un atome d'hydrogène ou de fluor.

5. Composés selon la revendication 4, caractérisés en ce que les deux substituants Y¹ et Y² représentent des atomes d'hydrogène.

6. Composés selon l'une des revendications 1 à 5, caractérisés en ce que X représente un groupe alkyle ayant de 1 à 3 atomes de carbone, et X¹ représente un atome de fluor ou de chlore, ou un groupe alkyle ayant de 1 à 3 atomes de carbone.

7. Composés selon l'une des revendications 1 à 5, caractérisés en ce que X représente un groupe alkyle ou alcényle ayant de 1 ou, respectivement, 2 à 3 atomes de carbone, -CH=CF₂, -CH=CHCl ou -CH=CHF.

8. Compositions de cristaux liquides contenant au moins deux composants, caractérisées en ce qu'au moins un composant est un composé de formule I défini dans la revendication 1.

9. Compositions de cristaux liquides selon la revendication 8, caractérisées en ce que la proportion de composés de formule I définis dans la revendication 1 va de 3 à 40 % en poids.

10. Compositions de cristaux liquides selon la revendication 8 ou 9, caractérisées en ce que la proportion de composés de formule I définis dans la revendication 1 va de 5 à 30 % en poids.

11. Utilisation des composés de formule I définies dans la revendication 1, á des fins électro-optiques.

12. Utilisation des compositions de cristaux liquides selon l'une des revendications 8 à 10, à des fins électro-optiques.
